# EUROPEAN PATENT APPLICATION

(11) **EP 4 462 127 A1**
(43) Date of publication of application: **13.11.2024**
(21) Application number: 22918742.2
(22) Date of filing: 29.11.2022
(51) Int. Cl.: G01N 35/00

(54) **PREDICTION LINE CALCULATING DEVICE, AND PREDICTION LINE CALCULATION METHOD**

(30) Priority: 05.01.2022 JP 2022000337
(71) Applicant: HITACHI HIGH-TECH CORPORATION, Tokyo 105-6409 (JP)
(72) Inventor: SASAKI Nobuhiko, Tokyo 105-6409 (JP); YABUTANI Chie, Tokyo 105-6409 (JP); IIJIMA Masahiko, Tokyo 105-6409 (JP)
(74) Representative: Strehl Schübel-Hopf & Partner
(86) International application number: PCT/JP2022/043869
(87) International publication number: WO 2023/132155

(57) **Abstract**

The objective of the present invention is to provide an automated analyzing device capable of obtaining information to be used for detecting an analysis abnormality in the course of an analysis step, without analyzing in advance a specimen having a known concentration. An automated analyzing device according to the present invention acquires a standard reaction process model from among a plurality of reaction processes, and generates a prediction line by applying a data point in the standard reaction process model to measurement result data of the reaction process

## Description

### Technical Field

The present invention relates to a technique for calculating a prediction line of a result measured by an automated analyzing device for analyzing a component contained in a specimen.

### Background Art

In an automated analyzing device for clinical examination, a specimen such as blood or urine is dispensed from a specimen container to a reaction container by a specimen aliquoting mechanism, and a reagent is dispensed from a reagent container to the reaction container dispensed with the specimen by a reagent aliquoting mechanism and is stirred. Thereafter, a concentration of a target item contained in the specimen is calculated by allowing the specimen to react with the reagent for a certain time and measuring an absorbance, a light emission amount, or the like obtained from a reaction liquid.

During the reaction between the specimen and the reagent, the absorbance or the light emission amount is stored in time series (for example, during 10 minutes, the absorbance or the light emission amount is acquired 38 times and stored in time series (hereinafter, referred to as a reaction process), and the presence or absence of an unintended change in a time series change is determined. When there is an unintended change, the automated analyzing device can notify a user that there is an abnormality in an analysis of the item.

PTL 1 discloses an automated analyzing device that determines a presence or absence of an abnormality from a deviation between an absorbance or a light emission amount acquired at an N-th time and an approximate curve applied in advance to a reaction process.

PTL 2 discloses an automated analyzing device that acquires a chemical reaction model from a specimen having a known concentration such as a quality control specimen and stores the chemical reaction model, calculates a reference reaction process from an absorbance change amount or an absorbance change rate of the specimen, and determines a presence or absence of an abnormality from a deviation between the reference reaction process and a reaction process of the specimen.

### Citation List

### Patent Literature

PTL 1: JP2010-271095A
PTL 2: JP2004-347385A

### Summary of Invention

### Technical Problem

In PTLs 1 and 2, the presence or absence of an abnormality cannot be determined unless the analysis is completed. To quickly determine the presence or absence of an abnormality, it can be said that it is desirable to obtain information that can be used for determining the presence or absence in an analysis process.

In PTL 2, it is necessary to prepare the chemical reaction model in advance based on the specimen having a known concentration. However, a time required to prepare the specimen having a known concentration, a time required to analyze the specimen having a known concentration, the cost of reagents, and the like are a burden on clinical examinations. Therefore, it can be said that it is desirable to obtain the information that can be used for determining the presence or absence of an abnormality without using the specimen having a known concentration.

The invention has been made in view of such problems, and an object thereof is to provide an automated analyzing device capable of obtaining information to be used for detecting an analysis abnormality in the course of an analysis step, without analyzing in advance a specimen having a known concentration.

### Solution to Problem

An automated analyzing device according to the invention acquires a standard reaction process model from among a plurality of reaction processes, and generates a prediction line by applying a data point in the standard reaction process model to measurement result data of the reaction process.

### Advantageous Effects of Invention

According to the automated analyzing device of the invention, it is possible to obtain the information to be used for detecting an analysis abnormality in the course of the analysis step, without analyzing in advance the specimen having a known concentration. Technical problems, configurations, effects, and the like other than those described above will become apparent in the following description of embodiments.

### Brief Description of Drawings

[FIG. 1] FIG. 1 is an overall configuration diagram of an automated analyzing device 100 according to Embodiment 1.
[FIG. 2] FIG. 2 is a schematic diagram showing a connection relationship between the automated analyzing device 100 and a server 200.
[FIG. 3] FIG. 3 shows an example of a standard reaction process model.
[FIG. 4] FIG. 4 is a flowchart showing a procedure for a calculation unit 210 to create the standard reaction process model.
[FIG. 5] FIG. 5 is a diagram showing a procedure for creating a prediction line in a reaction process.
[FIG. 6] FIG. 6 is a diagram showing a result of creating the prediction line by using abnormal measurement result data.
[FIG. 7] FIG. 7 is a graph showing a result obtained by overwriting prediction lines created by using normal measurement result data on the measurement result data.
[FIG. 8] FIG. 8 is a graph showing a result obtained by overwriting prediction lines created by using abnormal measurement result data on the measurement result data.
[FIG. 9A] FIG. 9A is a flowchart showing a procedure for the calculation unit 210 to determine the accuracy of the measurement result data by using the prediction line.
[FIG. 9B] FIG. 9B is a flowchart showing the procedure for the calculation unit 210 to determine the accuracy of the measurement result data by using the prediction line.
[FIG. 10] FIG. 10 is an example of plotting a temporal change of a deviation degree in S909.
[FIG. 11] FIG. 11 shows standard reaction process models of two measurement items.
[FIG. 12A] FIG. 12Ais a graph showing a temporal change in a moisture content when a subject A uses a sample of a skin care product A.
[FIG. 12B] FIG. 12B is a graph showing a temporal change in a moisture content when a subject B uses a sample of the skin care product A.

### Description of Embodiments

### <Embodiment 1>

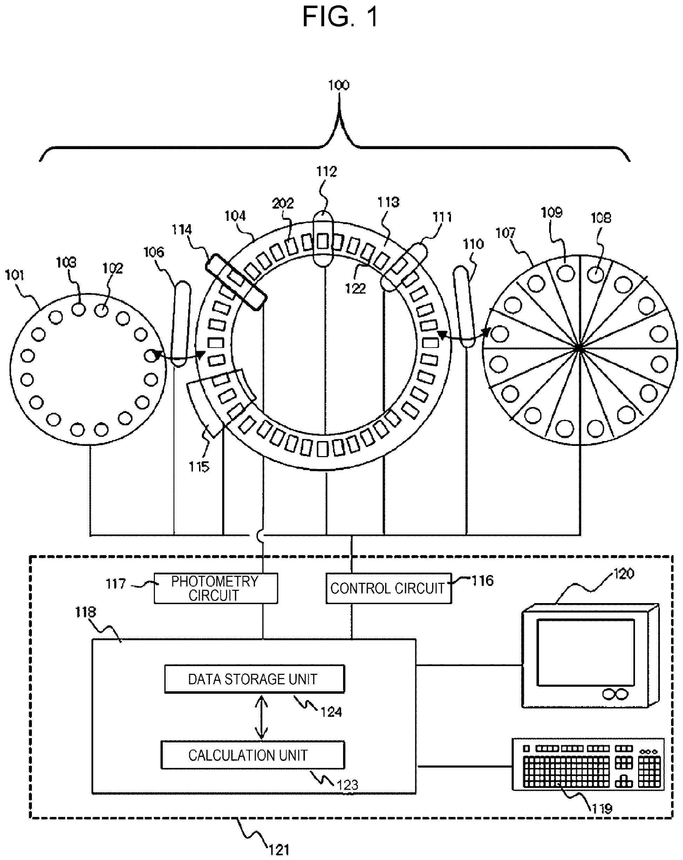

FIG. 1 is an overall configuration diagram of an automated analyzing device 100 according to Embodiment 1 of the present disclosure. The automated analyzing device 100 mainly includes a specimen disk 101, a reaction disk 104, a specimen aliquoting mechanism 106, a reagent disk 107, a reagent aliquoting mechanism 110, a sound wave irradiation mechanism 111, a stirring mechanism 112, a photometry mechanism 114, a reaction container cleaning mechanism 115, and an overall control unit 121. A specimen container 103 for accommodating a specimen 102 is disposed concentrically on the specimen disk 101. A reaction container 202 is disposed concentrically on the reaction disk 104, and the disposed reaction container 202 is maintained at a constant temperature by a constant temperature bath circulating liquid 113. A reagent container 109 for accommodating various reagents 108 is disposed concentrically on the reagent disk 107.

The overall control unit 121 includes a control circuit 116, a photometry circuit 117, and a computer 118, and an input unit 119 (for example, a pointing device or a keyboard) and an output unit 120 for displaying a measurement result and a graphical user interface (GUI) related to various operations are connected to the computer 118. In the example in FIG. 1, the control circuit 116 of the overall control unit 121 is connected to each configuration unit to control the entire device, and an independent control unit for each configuration unit may be provided.

An analysis by the automated analyzing device 100 is mainly executed as follows. First, the specimen 102 provided in the specimen disk 101 is dispensed from the specimen container 103 to the reaction container 202 on the reaction disk 104 by the specimen aliquoting mechanism 106. Thereafter, the reaction container 202 in which the specimen 102 is accommodated moves to a reagent aliquoting position by a rotation operation of the reaction disk 104, and the reagent aliquoting mechanism 110 dispenses the reagent 108 to be used for the analysis from the reagent container 109 to the reaction container 202 containing the specimen 102. A mixed liquid of the specimen 102 and the reagent 108 accommodated in the reaction container 202 is referred to as a reaction liquid 122. Subsequently, after the reaction liquid 122 in the reaction container 202 is degassed by the sound wave irradiation mechanism 111, the reaction liquid 122 in the reaction container 202 is stirred by the stirring mechanism 112. The reaction container 202 is maintained at a constant temperature, for example, 37 °C by the constant temperature bath circulating liquid 113 filled in a lower portion of the reaction disk 104, aimed at promoting the reaction and stabilizing the progress of the reaction.

When the reaction liquid 122 in the reaction container 202 passes through the photometry mechanism 114 as the rotation operation of the reaction disk 104, an optical characteristic change thereof is measured by the photometry circuit 117. The photometry data obtained in this way is transmitted to the computer 118, a concentration of a target component in the specimen is obtained by a calculation unit 123 in the computer 118, the obtained data is stored in a data storage unit 124, and a result is displayed on the output unit 120. The reaction container 202 after the reaction is cleaned by the reaction container cleaning mechanism 115, and is repeatedly used for the next reaction or is discarded to a reaction container disposal unit (not shown).

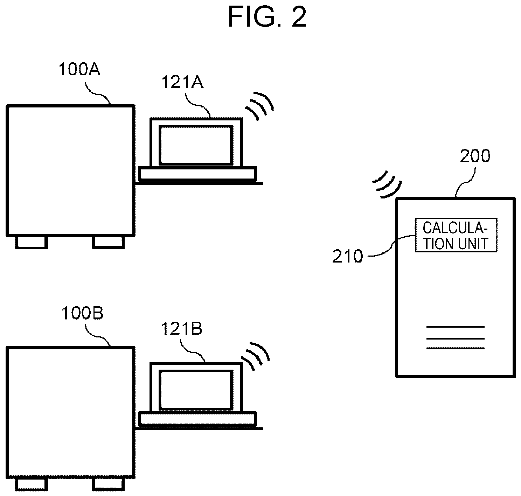

FIG. 2 is a schematic diagram showing a connection relationship between the automated analyzing device 100 and a server 200. Since there may be a plurality of the automated analyzing devices 100, the plurality of automated analyzing devices 100 are distinguished by alphabetic subscripts in FIG. 2. An automated analyzing device 100A transmits measurement result data (data describing a result obtained by measuring the reaction process of the specimen) to the server 200 via an overall control unit 121A, and an automated analyzing device 100B transmits the measurement result data to the server 200 via an overall control unit 121B.

The server 200 (prediction line calculating device) accumulates the measurement result data collected from the automated analyzing device 100. It is sufficient that the measurement result data includes all (for example, 38 times) of an absorbance or a light emission amount acquired in one analysis, a measurement item, a production lot number of the reagent, a concentration value of the measurement result, the presence or absence of an abnormality (data alarm) recognizable by the existing automated analyzing device 100, and the like, and additional information such as patient information is not necessary.

A calculation unit 210 included in the server 200 uses the accumulated measurement result data to determine the accuracy of the measurement result data by performing a procedure to be described later. The calculation unit 210 may be implemented by hardware such as a circuit device on which a function thereof is implemented, or may be implemented by a calculation device such as a central processing unit (CPU) executing software on which the function thereof is implemented.

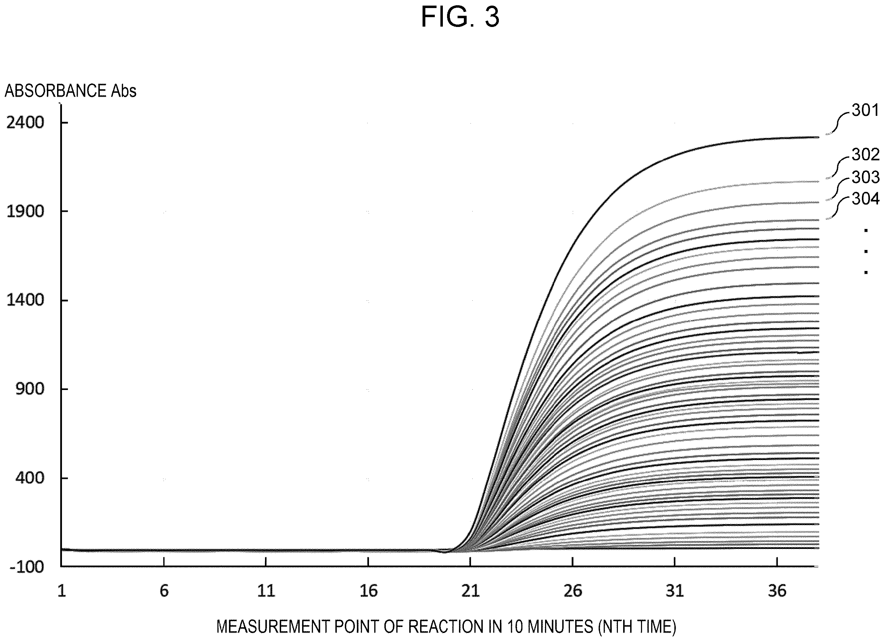

FIG. 3 shows examples of a standard reaction process model. The standard reaction process model is data describing a measurement result obtained in a standard reaction process of the specimen. The calculation unit 210 generates the standard reaction process model for each concentration of a specimen component according to the procedure to be described later. FIG. 3 shows four standard reaction process models 301 to 304.

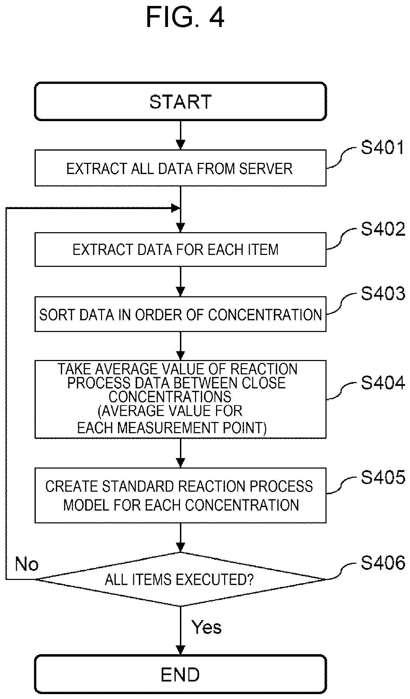

FIG. 4 is a flowchart showing a procedure for the calculation unit 210 to create the standard reaction process model. The present flowchart can be implemented, for example, at a monthly model update event. Hereinafter, each step in FIG. 4 will be described.

### (FIG. 4: Steps S401 to S403)

The calculation unit 210 acquires the measurement result data accumulated by the server 200 (S401). The calculation unit 210 extracts the reaction process (measurement result in time series) for each measurement item describing the measurement result data (S402). The calculation unit 210 aligns the measurement result data for, for example, each concentration of the specimen component (S403). For example, about 100 pieces of the measurement result data are associated with one specimen concentration.

### (FIG. 4: Step S404)

The calculation unit 210 calculates an average value of the measurement result data at a close specimen concentration. For example, it is assumed that 100 pieces of reaction process data classified as a concentration A are collected. As shown in FIG. 3, each reaction process is described, for example, starting from a first absorbance measurement result to a 38th absorbance measurement result. The calculation unit 210 averages values of 100 of the reaction processes as a first measurement result. Similarly, in the second to 38th measurement results, 100 of the reaction processes are averaged. Accordingly, an averaged reaction process is obtained for each concentration of the specimen component.

### (FIG. 4: Step S404: Supplement)

When there is a deviation equal to or greater than a threshold between the reaction process obtained by the averaging processing and the measurement result data, the calculation unit 210 may exclude the measurement result data from a target of the averaging processing as an outlier. The threshold is, for example, a standard deviation that can be calculated when averaging is performed. The calculation unit 210 may perform the averaging processing again on a measurement result data group excluding the outlier. Accordingly, an appropriate average value (that is, a more appropriate standard reaction process model) can be obtained.

### (FIG. 4: Step S405)

The calculation unit 210 stores the reaction process for each concentration obtained as a result of the averaging processing in S404 as the standard reaction process model. For example, a standard reaction process model of the concentration A is a standard reaction process model 301 shown in FIG. 3. Similarly, standard reaction process models are stored for a concentration B (standard reaction process model 302), a concentration C (standard reaction process model 303), a concentration D (standard reaction process model 304), ...

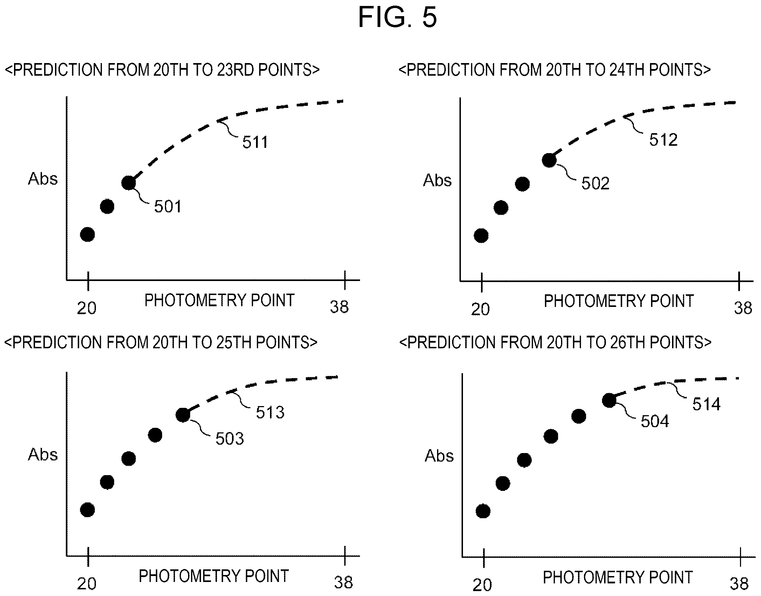

FIG. 5 is a diagram showing a procedure for creating a prediction line of the reaction process. When the measurement result data is normal, a temporal change in the measurement results after a certain measurement point is predicted to be similar to that of the standard reaction process model. The calculation unit 210 creates the prediction line of the reaction process by connecting data points of measurement results within a certain measurement point range with data points of the standard reaction process model at measurement points after the measurement points. In the example shown in FIG. 5, a procedure for creating prediction lines for the 20th and subsequent measurement points is shown.

The calculation unit 210 specifies a standard reaction process model that most matches a data point 501 among the 20th to 23rd points. The standard reaction process model is provisionally referred to as a standard reaction process model 1. The calculation unit 210 creates a prediction line 511 by connecting data points from the 24th to 38th points of the standard reaction process model 1 to the data point 501.

The calculation unit 210 specifies a standard reaction process model that most matches a data point 502 among the 20th to 24th points. The standard reaction process model is provisionally referred to as a standard reaction process model 2. The calculation unit 210 creates a prediction line 512 by connecting data points from the 25th to 38th points of the standard reaction process model 2 to the data point 502.

The calculation unit 210 specifies a standard reaction process model that most matches a data point 503 among the 20th to 25th points. The standard reaction process model is provisionally referred to as a standard reaction process model 3. The calculation unit 210 creates a prediction line 513 by connecting data points from the 26th to 38th points of the standard reaction process model 3 to the data point 503.

The calculation unit 210 specifies a standard reaction process model that most matches a data point 504 among the 20th to 26th points. The standard reaction process model is provisionally referred to as a standard reaction process model 4. The calculation unit 210 creates a prediction line 514 by connecting data points from the 27th to 38th points of the standard reaction process model 4 to the data point 504.

When the prediction line is created by the procedure described above on the normal measurement result data, all of the standard reaction process models 1 to 4 are the same, and the prediction lines 511 to 514 are also substantially the same reaction process.

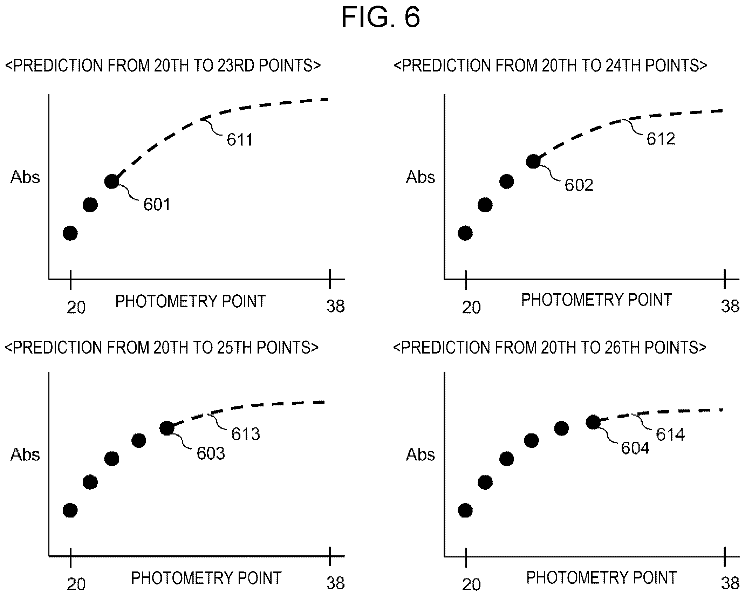

FIG. 6 is a diagram showing a result of creating the prediction lines by using abnormal measurement result data. In the example, the calculation unit 210 creates the following prediction lines as in FIG. 5: (a) creating a prediction line 611 for a data point 601; (b) creating a prediction line 612 for a data point 602; (c) creating a prediction line 613 for a data point 603; and (d) creating a prediction line 614 for a data point 604.

When the prediction line is created by the procedure described above based on the abnormal measurement result data, at least one of the standard reaction process models 1 to 4 is different from the other standard reaction process models, and at least one of the prediction lines 611 to 614 is a reaction process different from the other prediction lines.

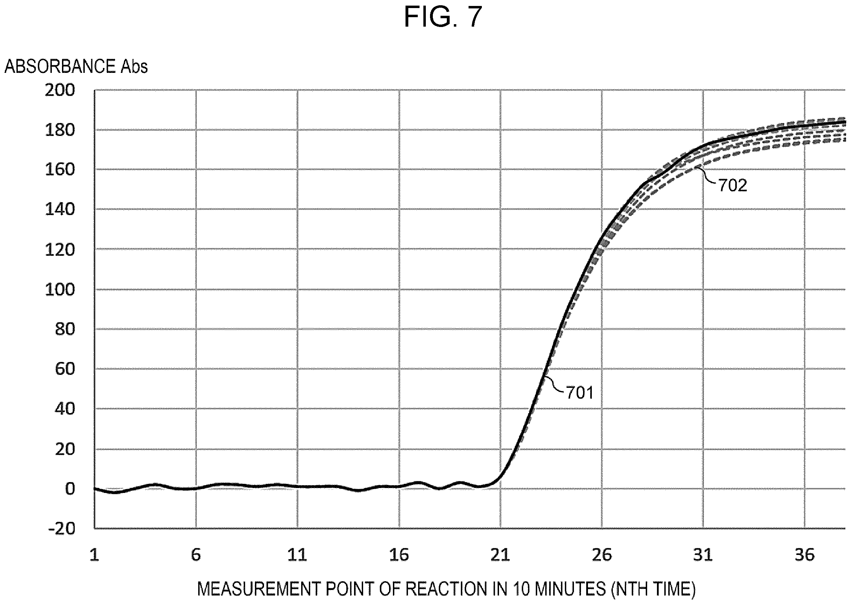

FIG. 7 is a graph showing a result obtained by overwriting the prediction lines created by using the normal measurement result data on the measurement result data. Measurement result data 701 (solid line) indicates an actual measurement result. A prediction line 702 (dotted line) is each of the prediction lines created by the procedure described with reference to FIGS. 5 and 6. The calculation unit 210 presents a result obtained by overwriting the measurement result data 701 and the prediction lines 702 on one graph. For example, a presentation format may be a display of a screen on a display included in the server 200 or may be an output of data describing the overwritten graph to an appropriate medium. It can be understood that the prediction lines created by using the normal measurement result data are gathered within a narrow range as shown in FIG. 7.

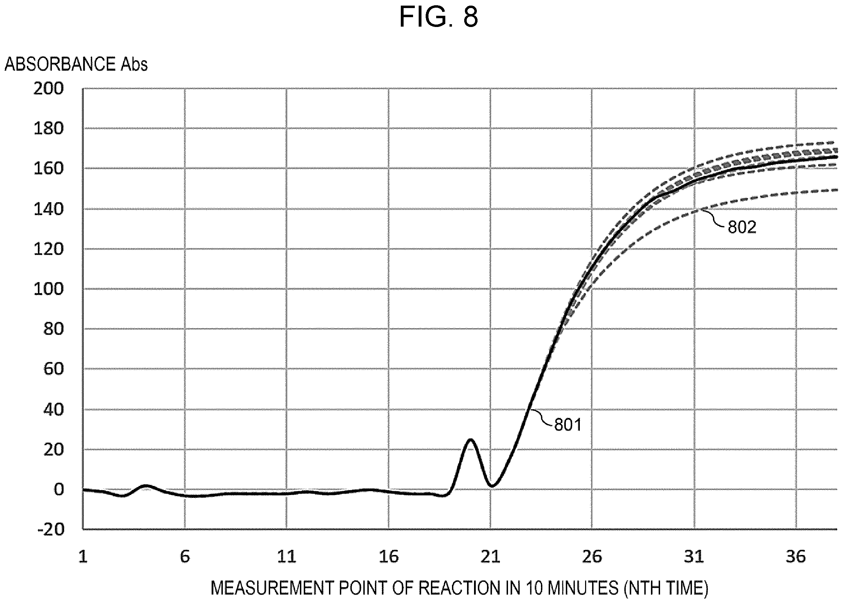

FIG. 8 is a graph showing a result obtained by overwriting the prediction lines created by using the abnormal measurement result data on the measurement result data. It can be understood that the prediction lines created by using the abnormal measurement result data are scattered in a wide range as shown in FIG. 8.

As shown in FIGS. 7 and 8, it is possible to evaluate whether the measurement result data is normal based on whether the prediction lines are gathered within a narrow range (that is, the prediction lines represent substantially the same reaction process) or scattered in a wide range (that is, the prediction lines represent different reaction processes). Further, as shown in FIGS. 7 and 8, the evaluation can be visually performed by the overwriting.

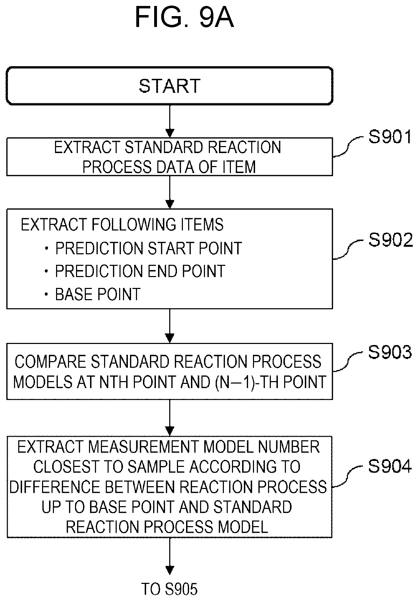

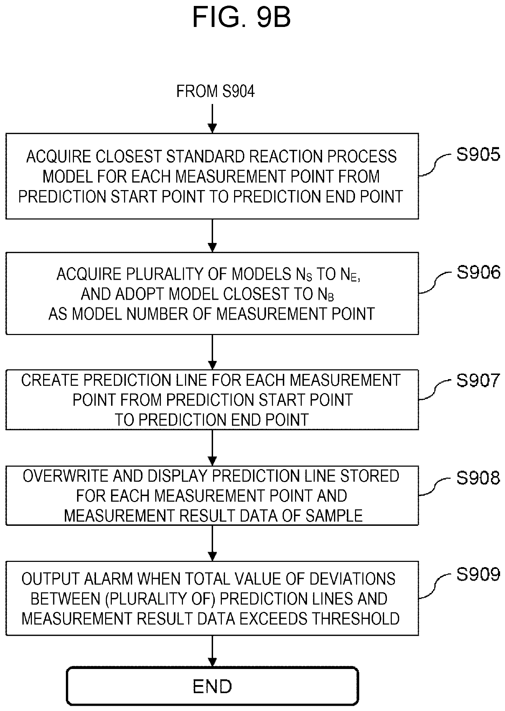

FIGS. 9A and 9B are flowcharts showing a procedure for the calculation unit 210 to determine the accuracy of the measurement result data by using the prediction line. The present flowchart can be executed to determine the accuracy of the measurement result data after the automated analyzing device 100 acquires the measurement result data of the actual sample. Hereinafter, each step in FIG. 9A will be described.

### (FIG. 9A: Step S901)

The calculation unit 210 acquires the measurement result data of the sample from the automated analyzing device 100. The calculation unit 210 extracts standard reaction process data for each measurement item. The measurement item corresponds to, for example, a component contained in the specimen.

### (FIG. 9A: Step S902)

The calculation unit 210 sets any one of the data points in the measurement result data as (a) a prediction start point, (b) a prediction end point, and (c) a base point. Which data point is used as these points can be determined in advance.

### (FIG. 9A: Step S902: Supplement)

The prediction start point is a start point of the data point of the measurement result data to be compared when the prediction line is created and is the 20th point in the examples of FIGS. 7 and 8. The prediction end point is an end point of the data point of the measurement result data to be compared when the prediction line is created, and is, for example, the 30th point. When it is known in advance that a correlation between the concentration and a measurement value is low at the first to 19th points, these data points are unnecessary when creating the prediction line. Similarly, when the correlation is low at the 31st to 38th points, these data points are unnecessary. To exclude these data points, the prediction start point and the prediction end point are set. The base point is, for example, the 23rd point. The base point is used in a step to be described later.

### (FIG. 9A: Step S903)

The calculation unit 210 compares the data point in the measurement result with the standard reaction process model. A comparison procedure is the same as that described with reference to FIGS. 5 and 6. However, as to be described below, a simplified comparison procedure can be used.

### (FIG. 9A: Step S903: Supplement 1)

In the examples described with reference to FIGS. 5 and 6, it has been described that the data point to be compared with the standard reaction process model is gradually increased using the 20th point as the start point, and the data point to be compared with the standard reaction process model is not limited thereto. For example, only the most recent points may be compared with the standard reaction process model. For example, the calculation unit 210 may compare the latest measurement value of an Nth point and the measurement value of an (N-1)-th point immediately before the Nth point with the standard reaction process model. FIG. 9A shows such an example.

### (FIG. 9A: Step S903: Supplement 2)

However, when the number of data points to be compared with the standard reaction process model is reduced, an accuracy of matching with the standard reaction process model may decrease, and the plurality of standard reaction process models may be extracted as matching candidates. In such a case, it is sufficient that a standard reaction process model having the highest accuracy of matching may be selected based on the following S904 to S906.

### (FIG. 9A: Step S904)

The calculation unit 210 specifies a number of a standard reaction process model closest to the sample (called as a model N_{B}) according to a difference between the reaction process from the first point to the base point (for example, the 23rd point) and the standard reaction process model. A reason why the standard reaction process model is specified based on the reaction process up to the base point is that it is known in advance that the measurement result up to the base point has the highest correlation with the standard reaction process model. N_{B} is used in correction processing to be described later.

### (FIG. 9B: Step S905)

The calculation unit 210 specifies a standard reaction process model Ns that most matches the measurement value from the first point to the prediction start point (the 20th point). This corresponds to specifying the standard reaction process model 1 described with reference to FIGS. 5 and 6. Similarly, the calculation unit 210 specifies a standard reaction process model that most matches the measurement values from the first to 21st points, a standard reaction process model that most matches the measurement values from the first to 22nd points, and a standard reaction process model N_{E} that most matches the measurement values from the first point to the prediction end point (30th point).

### (FIG. 9B: Step S906)

The calculation unit 210 can specify one or more of each of the models Ns to N_{E} in S905. When a plurality of models are specified, a model that most matches the measurement result data among the plurality of models is specified. Specifically, the models that most match the model N_{B} are specified as the models Ns to N_{E}, respectively.

### (FIG. 9B: Step S907)

The calculation unit 210 creates the prediction line for each measurement point from the prediction start point to the prediction end point. Specifically, the prediction line is obtained by executing the procedure described with reference to FIG. 5 (or the simplified procedure described in S903: Supplementary) at the prediction start point to the prediction end point. The present step corresponds to creating the prediction lines described with reference to FIGS. 5 and 6.

### (FIG. 9B: Step S908)

The calculation unit 210 overwrites the prediction line for each measurement point and the reaction process of the first to 38th points of the sample on one screen. The present step corresponds to creating the overwritten graph, described with reference to FIGS. 7 and 8.

### (FIG. 9B: Step S909)

The calculation unit 210 sums up (a) a deviation amount between the prediction lines for each measurement point and (b) a deviation amount between the data point of the 38th point in the reaction process of the sample and the prediction line, and calculates a total value thereof as a reaction accuracy of the sample. When the reaction accuracy exceeds a threshold, an alarm for notifying that the sample is abnormal is output.

### (FIG. 9B: Step S909: Supplement)

In the present step, the deviation between the standard reaction process model and the prediction line can be used as a threshold in the present step. For example, the threshold may be three times the standard deviation.

### <Embodiment 1: Summary>

The server 200 (prediction line calculating device) according to Embodiment 1 collects a plurality of pieces of reaction process data and acquires the standard reaction process model by using (for example, by averaging) the plurality of pieces of reaction process data, and determines the accuracy of the measurement result data by using the standard reaction process model. Therefore, the accuracy of the measurement result data can be determined without preparing in advance a specific specimen such as the specimen having a known concentration. Accordingly, a burden on a manufacturer side for preparing a specific specimen can be reduced.

In the process of acquiring the data point of the measurement result data, the server 200 according to Embodiment 1 can generate the prediction line by connecting the data point of the standard reaction process model to the data point, and can transmit an alert at a time point when the deviation degree between the prediction line and the measurement result data exceeds the threshold. Therefore, the presence or absence of an abnormality can be determined in the course of the reaction process before the analysis is completed. Accordingly, the user can early prepare a retest.

### <Embodiment 2>

In Embodiment 2 according to the invention, a specific example of S909 will be described. Other configurations are the same as those of Embodiment 1.

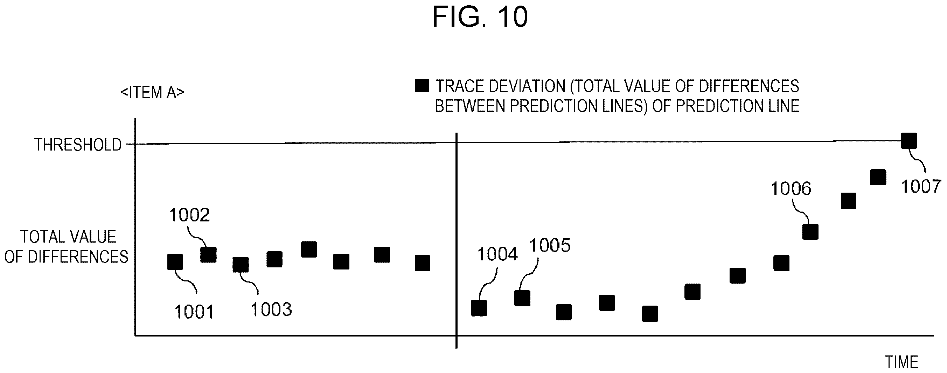

FIG. 10 is an example of plotting the temporal change of the deviation degree in S909. Plots 1001 to 1003 represent the reaction accuracies (totals of the deviation degrees of (a) and (b) calculated in S909) when samples 1 to 3 are analyzed in a reagent lot 1 for an item A. Plots 1004 to 1007 represent reaction accuracies when samples 4 to 1 are analyzed in a reagent lot 2 for the item A.

A temporal variation in the reaction accuracies of the plots 1001 to 1003 is small, whereas the reaction accuracy of the plot 1004 changed from the reagent lot 1 to the reagent lot 2 has a large deviation from the reaction accuracy of the plot 1003. If the deviation is small, it is suggested that a difference in the reagent component is small even when the reagent lot is changed, and therefore it can be said that there is no need to perform calibration again. On the other hand, if the deviation is large, it is suggested that there is a difference in the reagent component when the reagent lot is changed, and therefore it is desirable to perform the calibration again. Therefore, the calculation unit 210 recommends the user to perform the calibration.

As indicated by the plot 1007, when the reaction accuracy exceeds the threshold, the calculation unit 210 suggests to the user a possibility of reagent deterioration and recommends the user to replace the reagent.

When an item in which the reaction accuracy exceeds the threshold is not only the item A and there is a plurality of items in which the reaction accuracy exceeds the threshold, the calculation unit 210 recommends a replacement of a consumable item of the automated analyzing device 100 such as a light source or a detector.

The calculation unit 210 can further predict the temporal change in the reaction accuracy in the future by fitting the plots shown in FIG. 10. Accordingly, a timing at which the reaction accuracy exceeds the threshold can be predicted.

### <Embodiment 2: Summary>

The server 200 according to Embodiment 2 calculates the accuracy of the measurement result data by summing up the deviation degree between the prediction lines and the deviation degree between the prediction line and the measurement result data, and determines the presence or absence of an abnormality in the measurement result data according to the temporal change in the accuracy. Accordingly, it is possible to determine the accuracy of the measurement result without requiring time to prepare a specimen having a known concentration, time required for analyzing the specimen having a known concentration, cost of the reagent, and the like for accuracy control in advance.

The server 200 according to Embodiment 2 can prompt the calibration when the reaction accuracy does not exceed the threshold but the temporal change plot is rapidly changed (for example, the plot 1004 in FIG. 10, but a difference between the plot 1004 and a plot immediately before the plot 1004 is smaller than the threshold on a vertical axis in FIG. 10). A possibility of reagent deterioration can be suggested if there is one measurement item in which the reaction accuracy exceeds the threshold. A replacement of a consumable part of the automated analyzing device 100 can be prompted if there are two or more measurement items in which the reaction accuracy exceeds the threshold.

In the server 200 according to Embodiment 2, a content of the alert is determined according to the number of items in which the reaction accuracy exceeds the threshold, and the number can be freely set. Therefore, it should be noted that the numerical values described above regarding the number are one example. No matter how the number is changed, it's the same that a large number leads to a prompt of replacement of the consumables, while a small number leads to a suggest of reagent deterioration.

### <Embodiment 3>

In Embodiment 3 according to the invention, an example will be described in which a moisture content of human skin is analyzed as an example of a specimen component analyzed by the automated analyzing device 100. A configuration of the automated analyzing device 100 is the same as that in Embodiments 1 and 2.

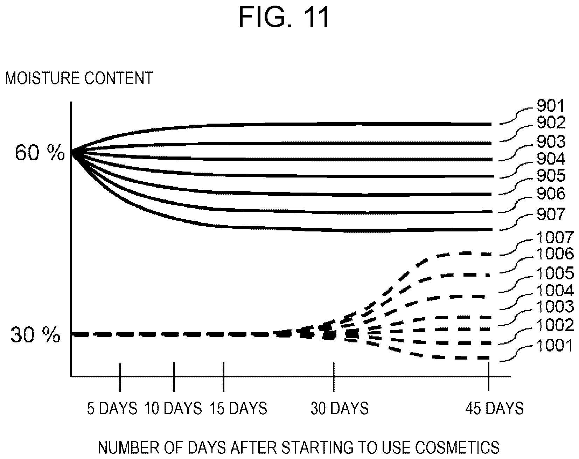

FIG. 11 shows standard reaction process models of two measurement items. A model 901 indicates a moisture content change of a stratum basale inside the skin of a skin model 1. Similarly, models 902 to 907 show moisture content changes of the stratum basale of skin models 2 to 7. Models 1001 to 1007 indicate moisture content changes of a stratum corneum on a skin surface of the skin models 1 to 7. Therefore, the model 901 and the model 1001 are associated with each other, and similarly, the models 902 to 907 and the models 1002 to 1007 are associated with one another.

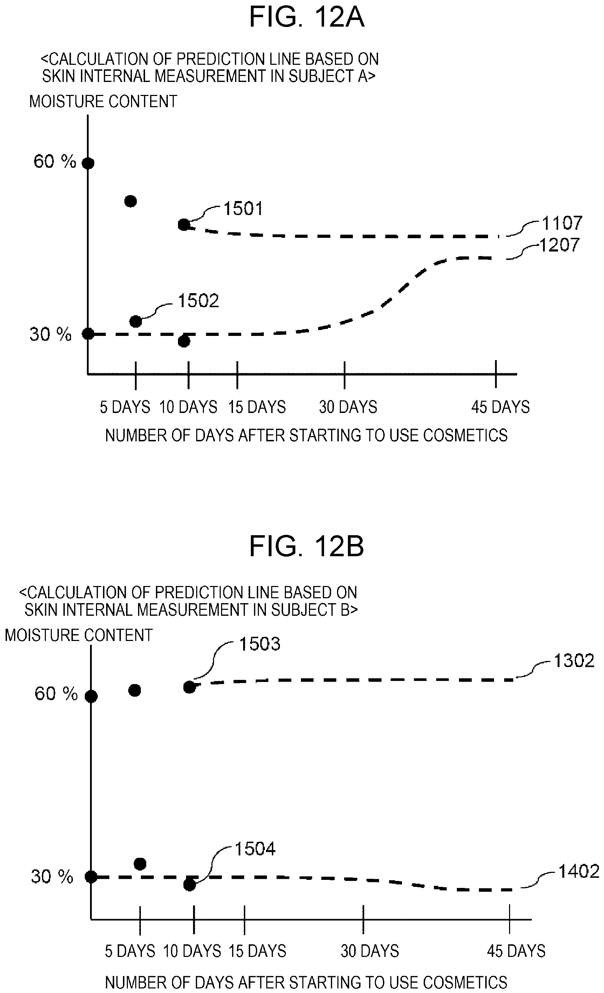

FIG. 12Ais a graph showing a temporal change in the moisture content when a subject A uses a sample of a skin care product A. In this example, measurement results are shown at the start of use, on the 5th day from the start of use, and on the 10th day from the start of use. A plot 1501 is a result obtained by measuring the moisture content of the stratum basale inside the skin. A plot 1502 is a result obtained by measuring the moisture content of the stratum corneum on the skin surface.

The calculation unit 210 specifies a model closet to the plot 1501 among the models 901 to 907 of the moisture content change of the stratum basale inside the skin. Here, it is assumed that the model 907 is the closest. The calculation unit 210 creates a prediction line 1107 by connecting the data points of the model 907 after the 10th day to the plot 1501. Similarly, the calculation unit 210 creates a prediction line 1207 by connecting the data points of the model 1007 after the 10th day corresponding to the model 907 to the plot 1502.

If linking data indicating that a skin type of the skin model 7 has a good compatibility with the skin care product A in terms of usage effects is created in advance, the calculation unit 210 can recommend that the subject A purchase the skin care product A at a time point at which the subject A uses the sample of the skin care product A for 10 days. The calculation unit 210 can indicate to the subject A that the state of the stratum corneum on the skin surface is predicted to be improved after one month with continued use of the skin care product A, and can indicate a basis for recommending that the skin care product A be purchased. If the subject A can actually feel that the state of the stratum corneum on the skin surface is improved after one month by the subject A actually using the skin care product A continuously, the reliability of the prediction described above can be indicated.

FIG. 12B is a graph showing a temporal change in the moisture content when a subject B uses a sample of the skin care product A. In this example, as in FIG. 12A, measurement results at the start of use, on the 5th day, and on the 10th are shown. A plot 1503 is a result obtained by measuring the moisture content of the stratum basale inside the skin. A plot 1504 is a result obtained by measuring the moisture content of the stratum corneum on the skin surface.

The calculation unit 210 specifies a model that most matches the plot 1503 among the models 901 to 907. Here, it is assumed that the model 902 is the closest. The calculation unit 210 creates a prediction line 1302 by connecting the data points of the model 902 after the 10th day to the plot 1503. Similarly, the calculation unit 210 creates a prediction line 1402 by connecting the data points of the model 1002 after the 10th day corresponding to the model 902 to the plot 1504.

If linking data is created in advance that indicates that a skin type of the skin model 2 has a poor compatibility with the skin care product A in terms of usage effects, but has a good compatibility with the skin care product B in terms of usage effects, the calculation unit 210 can suggest to the subject B, instead of the skin care product A, that use or purchase of the sample of the skin care product B which has a good compatibility in terms of usage effects at a time point at which the subject B uses the sample of the skin care product A for 10 days. The calculation unit 210 can indicate to the subject B that the state of the stratum corneum on the skin surface is predicted to improve after one month with continued use of the skin care product B, and can indicate a basis for recommending that the skin care product B be purchased. If the subject B can actually feel that the state of the stratum corneum on the skin surface is improved after one month by the subject B actually using the skin care product B continuously, the reliability of the prediction described above can be indicated.

As in Embodiment 1, the prediction line and the measurement result data can be overwritten on one screen. In this case, it can be expressed that if the deviation between the prediction lines is large, a prediction error is large, and if the deviation between the prediction lines is small, the prediction error is small.

The calculation unit 210 may calculate statistics of the probability that the measurement result data to be actually measured next enters between the prediction lines by using the data used when creating the standard reaction process model or the measurement result data that is actually measured. Accordingly, for example, at the time point at which the user uses the sample of the skin care product A for 10 days, the probability that the measurement result (moisture content) in the future falls between the prediction lines can be displayed at the same time as the overwritten display of the prediction lines.

### <Modifications of Invention>

The invention is not limited to the embodiments described above and includes various modifications. For example, the embodiments described above have been described in detail for easy understanding of the present disclosure, and are not necessarily limited to those having all the configurations described above. A part of a configuration according to one embodiment can also be replaced with a configuration according to another embodiment, and a configuration according to one embodiment can also be added to a configuration according to another embodiment. In addition, another configuration can be added to, deleted from, or replaced with a part of a configuration of each embodiment.

In the above-described embodiments, it is described that the server 200 determines the accuracy of the measurement result data acquired by the automated analyzing device 100, and the automated analyzing device 100 (for example, the overall control unit 121) may determine the accuracy of the measurement result data. In this case, the overall control unit 121 can be implemented similarly to the calculation unit 210, and the automated analyzing device 100 serves as a prediction line calculating device.

In the embodiments described above, it is described that the server 200 collects the measurement result data through communication from the automated analyzing device 100, and a method of collecting the measurement result data is not limited thereto. Instead of measuring the reaction process of the specimen, the reaction process of the sample having a known concentration may be collected. Further, an ideal reaction process may be generated and collected through a simulation or the like.

In the embodiments described above, a definition of explanatory variables and the method of specifying the closest standard reaction process model are presented as an example, and other definitions and methods can be used.

In Embodiment 3, a device for measuring the moisture content of the skin of a person is not necessarily the automated analyzing device 100 described in Embodiment 1. As long as a measurement result can be obtained and analyzed from a device capable of measuring at least the moisture content of the skin of the person as a measurement target, the invention is applicable to the measurement result.

### Reference Signs List

100: automated analyzing device
200: server
210: calculation unit

## Claims

1. A prediction line calculating device for calculating a prediction line of a measurement result obtained by an automated analyzing device configured to analyze a component contained in a specimen performing measurement,
the automated analyzing device measuring a reaction process of the specimen along a time series,
the prediction line calculating device comprising:
a calculation unit configured to calculate a prediction line of measurement result data describing a result obtained by the automated analyzing device performing measurement, wherein
the calculation unit acquires a standard reaction process model representing a standard reaction process generated from a plurality of the reaction processes,
the calculation unit generates a first prediction line by adding a data point after a first time point in the reaction process in the standard reaction process model to a data point of the measurement result data at the first time point,
the calculation unit generates one or more second prediction lines by adding, to a data point of the measurement result data at each of one or more subsequent time points after the first time point in the reaction process, a data point after the subsequent time point in the standard reaction process model, and
the calculation unit outputs the first prediction line and the second prediction line.

2. The prediction line calculating device according to claim 1, wherein
the calculation unit determines accuracy of the measurement result data according to a deviation degree between at least one of the first prediction line or the second prediction line and the measurement result data.

3. The prediction line calculating device according to claim 1, wherein
the calculation unit classifies the measurement result data in each of the plurality of reaction processes into one or more groups according to an attribute of the specimen,
the calculation unit creates the standard reaction process model for each of the groups by excluding a statistical outlier for the corresponding group, and
the calculation unit generates the first prediction line and the second prediction line for each of the groups by using the standard reaction process model for the corresponding group.

4. The prediction line calculating device according to claim 1, wherein
the calculation unit acquires the standard reaction process model for each attribute of the specimen,
the calculation unit specifies the standard reaction process model that matches the reaction process from a start time point before the first time point in the reaction process to the first time point as a first model,
the calculation unit generates the first prediction line by using the first model,
the calculation unit specifies the standard reaction process model that matches the reaction process from the start time point to the subsequent time point as a second model, and
the calculation unit generates the second prediction line by using the second model.

5. The prediction line calculating device according to claim 4, wherein
the calculation unit specifies the standard reaction process model that most matches the reaction process from the start time point of the reaction process to a reference time point in the reaction process as a reference model,
the calculation unit adopts a model closest to the reference model as the first model when there are a plurality of candidates of the first model, and
the calculation unit adopts a model closest to the reference model as the second model when there are a plurality of candidates of the second model.

6. The prediction line calculating device according to claim 1, wherein
the calculation unit displays the measurement result data on a screen of a display device, and displays at least one of the first prediction line or the second prediction line on the same screen as the measurement result data in a superimposed manner.

7. The prediction line calculating device according to claim 1, wherein
the calculation unit calculates a deviation degree between at least one of the first prediction line or the second prediction line and the measurement result data by adding
a difference of at least one of the first prediction line or the second prediction line with respect to the measurement result data, and
a difference between the first prediction line and the second prediction line at a specific measurement time point in the reaction process, and
the calculation unit outputs an alert indicating that accuracy of the measurement result data does not reach a reference value when the deviation degree exceeds a first threshold.

8. The prediction line calculating device according to claim 7, wherein
the calculation unit acquires the standard reaction process model for each of measurement items of the specimen,
the calculation unit calculates the deviation degree for each of the measurement items, and
the calculation unit sets a content of the alert according to the number of the measurement items in which the deviation degree exceeds the first threshold.

9. The prediction line calculating device according to claim 8, wherein
the calculation unit sets the alert for prompting execution of a calibration of the automated analyzing device when a temporal change amount of the deviation degree exceeds a second threshold smaller than the first threshold,
the calculation unit sets the alert for prompting a replacement of a reagent to be reacted with the specimen when the number of the measurement items in which the deviation degree exceeds the first threshold exceeds a first number and is equal to or less than a second number, and
the calculation unit sets the alert for prompting a replacement of a consumable part of the automated analyzing device when the number of the measurement items in which the deviation degree exceeds the first threshold exceeds the second number.

10. The prediction line calculating device according to claim 1, wherein
the calculation unit acquires the standard reaction process model for each of measurement items of the specimen and for each of groups of the specimen,
the calculation unit specifies in advance an association between the standard reaction process model acquired for a first measurement item of a first specimen group and the standard reaction process model acquired for a second measurement item of the first specimen group,
the calculation unit generates the first prediction line and the second prediction line for the first measurement item by specifying the standard reaction process model that matches the measurement result data acquired for the first measurement item,
the calculation unit specifies, according to the association, the standard reaction process model for the second measurement item that corresponds to the standard reaction process model specified for the first measurement item, and
the calculation unit generates the first prediction line and the second prediction line for the second measurement item by using the standard reaction process model specified for the second measurement item.

11. The prediction line calculating device according to claim 10, wherein
the first measurement item is a first moisture content of a stratum basale inside a skin of a person,
the second measurement item is a second moisture content of a stratum corneum on a skin surface of the person,
the group of the specimen is a group of subjects who are similar in the number of days of using a skin care product and a relationship between changes in the first and second moisture contents, and
the calculation unit specifies the association by specifying a correspondence relationship between the standard reaction process model representing the change in the first moisture content and the standard reaction process model representing the change in the second moisture content for each of the groups of the subjects.

12. The prediction line calculating device according to claim 11, wherein
the calculation unit specifies in advance a correspondence relationship between the skin care product which is recommended, and the group of the subjects for at least one of the first measurement item or the second measurement item,
the calculation unit acquires the measurement result data for a specific subject who uses a specific skin care product, and generates the first prediction line and the second prediction line by using the measurement result data,
the calculation unit outputs data indicating a recommendation of the specific skin care product when the first prediction line and the second prediction line indicate a good result as a result of skin care, and
the calculation unit outputs data indicating the recommended skin care product according to the correspondence relationship between the recommended skin care product and the group of the subjects when the first prediction line and the second prediction line indicate a poor result as the result of the skin care.

13. The prediction line calculating device according to claim 10, wherein
the calculation unit displays the measurement result data on a screen of a display device, and displays the first prediction line and the second prediction line on the same screen as the measurement result data in a superimposed manner.

14. The prediction line calculating device according to claim 13, wherein
the calculation unit statistically calculates a probability that the measurement result data falls within a region formed by the first prediction line and the second prediction line, and
the calculation unit displays, together with the measurement result data, the first prediction line, and the second prediction line, a probability that the measurement result data acquired up to a specific time point falls within the region after the time point.

15. A prediction line calculation method for calculating a prediction line of a measurement result obtained by an automated analyzing device configured to analyze a component contained in a specimen,
the automated analyzing device measuring a reaction process of the specimen along a time series,
the prediction line calculation method comprising:
a step of calculating a prediction line of measurement result data describing a result obtained by the automated analyzing device performing measurement, wherein
in the step of calculating the prediction line, a standard reaction process model representing a standard reaction process generated from a plurality of the reaction processes is acquired,
in the step of calculating the prediction line, a first prediction line is generated by adding a data point after a first time point in the reaction process in the standard reaction process model to a data point of the measurement result data at the first time point,
in the step of calculating the prediction line, one or more second prediction lines are generated by adding, to a data point of the measurement result data at each of one or more subsequent time points after the first time point in the reaction process, a data point after the corresponding subsequent time point in the standard reaction process model, and
in the step of calculating the prediction line, the first prediction line and the second prediction line are output.
